(19) Europäisches Patentamt
European Patent Office
Office européen des brevets

(11) Publication number: **0 208 272 B1**

(12) # EUROPEAN PATENT SPECIFICATION

(45) Date of publication of patent specification: **06.03.91**

(51) Int. Cl.5: **C07D 303/40**, C07D 301/12, //B01J31/24

(21) Application number: **86109120.5**

(22) Date of filing: **03.07.86**

(54) **Method for the preparation of alkyl esters of 3,4-epoxybutyric acid.**

(30) Priority: **05.07.85 IT 2146485**

(43) Date of publication of application:
**14.01.87 Bulletin 87/03**

(45) Publication of the grant of the patent:
**06.03.91 Bulletin 91/10**

(84) Designated Contracting States:
**BE CH DE FR GB LI NL**

(56) References cited:
**EP-A- 0 109 273**

**BULLETIN DE LA SOCIETE CHIMIQUE DE FRANCE, 1960, pages 1114-1116, Paris, FR; R. RAMBAUD et al.: "Oxydation perbenzoique de dérivés crotoniques et vinylacétiques"**

(73) Proprietor: **TECNOPART S.r.l.**
**16, Piazza della Repubblica**
**Milan(IT)**

(72) Inventor: **Venturello, Carlo, Dr.**
**135, via XXIII Marzo**
**I-28100 Novara(IT)**
Inventor: **Coassolo, Alfredo**
**34, via Gorizia**
**I-28100 Novara(IT)**
Inventor: **D'Aloisio, Rino**
**25, via Romention**
**I-28100 Novara(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

EP 0 208 272 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for the preparation of alkyl esters of 3,4-epoxybutyric acid of formula:

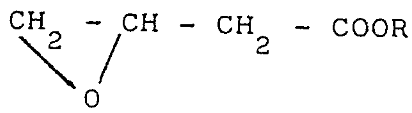
(I.)

wherein R is a linear or branched alkyl group containing from 4 to 10 C atoms, starting from the corresponding vinyl-acetates of formula:

$$CH_2 = CH - CH_2COOR \qquad (II)$$

werein R has the meaning given above.

Alkyl esters of 3,4-epoxybutyric acid (I) are an interesting class of compounds, endowed with peculiar features, which can be used as monomers and co-monomers in several kinds of polymerizations and co-polymerizations; furthermore they are important intermediates for sophisticated products endowed with biological and/or pharmaceutical activity.

Starting from alkyl esters of 3,4-epoxybutyric acid there may be obtained functional adenine derivatives, which are very useful in affinity chromatography or as macromolecular co-enzymes, not diffusable and water-soluble, these derivatives being obtained by allowing esters of epoxybutyric acid to react with adenine or derivatives thereof. Starting from the al kyl esters of 3,4-epoxybutyric acid it is possible to obtain the chloride of (R,S)-carnitine (and other analogous derivatives of carnitine). The importance of the R- (-) form thereof in the transfer of fatty acids through membranes is well known; carnitine catalyzes the oxidation of long-chain fatty acids and takes part in the transfer, by means of enzymes, of fatty acids activated by cytoplasm towards the $\beta$-oxidation sites within mitochondrias.

It is furthermore known to use the alkyl esters of 3,4-epoxy-butyric acid for the synthesis of derivatives of 2-oxo-1-pyrrolidine-acetamide, as described in EP-A-154 490; said derivatives are valuable psychotropic drugs which can restore the activity of cognitive processes impaired by different pathologic conditions.

The preparation of alkyl esters of 3,4-epoxybutyric acids, however,was hitherto been carried out with not very satisfactory results, with clearly negative influences at the industrial-economic level; these influences partially or totally make void the advantageous features of alkyl esters of 3,4-epoxybutyric acid. It is known to prepare esters of 3,4-epoxybutyric acid by oxidation of the corresponding vinylacetetes with peracids (like, for instance, peracetic or m-chloroperbenzoic acid in organic solvents; however, yields and long reaction times (4-10 days) ` as well as the cost of the oxidizing agents, which have to be used in at least stoichiometric quantity with respect of the substrate make this process of limited practical interest. There also is reported a synthesis of the methyl ester of 3,4-epoxybutyric acid starting from epichlorohydrin, by carbomethoxylation with carbon mono-oxide and methanol (in the presence of cobalt cabonyl), formation of 4-chloro-3-hydroxybutyric acid methyl ester and subsequent epoxide closure with silver oxide; however, this process is of no practical interest at the industrial level, because of drastic conditions, low yields and the high cost of some reactants.

EP-A-109273 describes peroxide compositions of formula $QXW_4O_{24-2n}$. Wherein Q represents a cation of an onium salt, X is P or As and n is 0, 1 or 2. Said compositions are reported to be suitable particularly as Catalysts for the epoxidation of olefinic compounds. The compounds actually oxidized in the presence of said peroxide compositions, however, are e.g. olefins, allylchloride and $\alpha$, $\beta$-unsaturated carboxylic acids.

There has now been found a simple and cheap method for the synthesis of alkyl esters of 3,4-epoxybutyric acid, free of the above mentioned drawbacks; this method provides products which, because of their purity level and low price (also resulting from the very high yields of the synthesis),are fairly good intermediates for the preparation of many chemical compounds endowed with biological and/or pharmaceutical activity.

In its broadest form the present invention relates to a method for the preparation of alkyl esters of 3,4-epoxybutyric acid of formula (I), wherein the corresponding vinylacetate of formula-(II) is catalytically oxidized, under phase transfer conditions, in a-biphasic liquid system comprising:

a) an aqueous phase, containing hydrogen peroxide;

b) an organic phase, containing said vinylacetate and a catalyst selected from the peroxidic compounds of formula: $(R_1\ R_2\ R_3\ R_4\ N)_3\ PW_4O_{24}$

as well as, optionally, a solvent immiscible with the aqueous phase, $R_1$, $R_2$, $R_3$ and $R_4$, the same or different from each other, being hydrocarbon groups, the total number of the carbon atoms of $R_1$, $R_2$, $R_3$ and $R_4$ ranging from 20 to 70 and preferably from 25 to 40.

The oxidation reaction can be represented as follows:

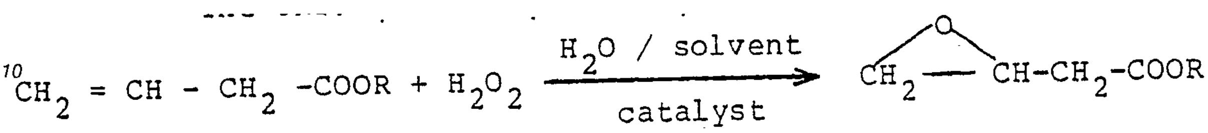


The epoxide is obtained within comparatively short periods of time (1 - 3 hrs), with high yields based on $H_2O_2$ (80-85%) and with a fairly good selectivity with respect to the vinylacetate (about 90%), with clear positive influences on the possible industrial application.

When the alkyl group R contains 4 to 10 carbon atoms the esters are obtained with better yields, R contains from 1 to 3 C atoms.

The best results were obtained when using a particular catalyst of formula:

$$(C_{25}H_{54}N)_3PW_4O_{24} \quad \text{(III)} \qquad \text{or} \qquad (C_{37}H_{78}N)_3PW_4O_{24} \quad \text{(IV)}$$

Catalysts (III) and (IV) consist of peroxidic compounds containing tungsten, P and a quaternary ammonium cation showing sufficient affinity for lipids, obtained according to usual techniques.

These catalysts, for instance, can be prepared by reacting a tungstic acid (or an alkaline tungstate), phosphoric acid (or an alkaline phosphate) and hydrogen peroxide in an acidic aqueous phase with a quaternary salt selected from methyltrioctylammonium chloride (known on the market as ALIQUAT® 336) and dimethyl [dioctadecyl (75%) + dihexadecyl (25%)] ammonium chloride (known on the market as ARQUAD® 2HT), contained in an organic phase immiscible with the aqueous phase. The reaction of the inorganic reactants can be carried out between 20° and 80°C; thereafter, the quaternary salt (dis solved in a solvent, for instance 1,2-dichloroethane, is added, preferably at room temperature, while continuing the stirring of the biphasic mixture for 15-30 minutes. The acidic phase had a pH lower than 2; in order to obtain said range of values, the pH is adjusted, if necessary, with a mineral acid (for instance $H_2SO_4$ or HCl). The molar ratios of the reactants must denerally be as follows: for 1 mole of P, 4 moles of W and up to 2 moles of quaternary salt are used; as to $H_2O_2$, from 2.5 to 6 moles of $H_2O_2$ are sufficient for 1 mole of W. After having separated the phases, by evaporating the organic phase, the compound (III) or (IV) is obtained in syrupy or solid form respectively.

The epoxidation reaction is carried out according to the double phase technique; the organic phase contains the vinylacetate, the catalyst and, optionally, a solvent immiscible with the aqueous phase. If a solvent is not used, a suitable excess of vinylacetate can be used. Chlorinated hydrocarbons (like, for instance, dichloroethanes, trichloroethane and tetrachloroethylene) or aromatic, optionally substituted, hydrocarbons (e.g. benzene, toluene or xylenes) can be used as immiscible solvents.

Generally, it is possible to work under strong stirring, at a temperature between 50° and 90°C, preferably between 65° and 75°C, under atmospheric pressure, and the reaction time (depending on the used catalyst and its amount, the working temperature and the concentration of the vinylacetate in the organic phase) generally between 1 and 3 hours; the catalyst is preferably used in a $H_2O_2$: catalyst molar ratio between 200 and 100.

Finally, it is advisable to work with a $H_2O_2$: substrate molar ratio between 1:1 and 1:2 and preferably from 1:1.5 to 1:1.6 when a solvent is used; the concentration of substrate (II) in the organic phase generally is between 30 and 90%, and preferably between 50 and 80% by weight. It is advisable to use, in the aqueous phase,a $H_2O_2$ concentration between 1 and 70%, and preferably between 10 and 30% by weight; thereby $H_2O_2$ conversions of 98-99% may be achieved.

At the end of the reaction, after having separated the phases, the product (present in the organic phase), may be separated from the catalyst (together with unreacted vinylacetate (II) and with the solvent)

by a quick pre-distillation under vacuum; the distillate then is fractionated under vacuum, thus obtaining product (I) in pure form; obviously, these are usual methods, allowing possible alternatives. The method according to the invention can be performed employing usual apparatus and expedients. The catalyst is sufficiently stable and, therefore, it can be prepared and stored until it is used. The invention will be further described in the following examples, given to merely illustrate the present invention, without limiting the scope thereof. The concentrations of hydrogen peroxide and phosphoric acid are expressed in the examples as grams per 100ml of water.

EXAMPLE 1

a) Preparation of the catalyst $(C_{37}H_{78}N)_3PW_4O_{24}$ Into a 100ml, four necked flask provided with blade stirrer and dropping funnel, there were introduced, at room temperature, 3.30 g of $Na_2WO_4.2H_2O$, dissolved in 20ml of water, 1.5ml of 40% $H_3PO_4$ and 3ml of 30% by weight $H_2SO_4$. Thereafter, 2ml of 40% $H_2O_2$ were added. 40ml of 1,2-dichloroethane containing 3.10 g of ARQUAD® 2HT were added, under stirring, over 2-3 minutes; ARQUAD® 2HT contains 75% of active substance. Stirring was continued for 20 minutes at room temperature and the phases were then decanted and separated.

The organic (lower) phase was evaporated, 3.6 g of a white solid being thus obtained; the analytical data were in agreement with the indicated formula, as pointed out hereinafter:

ACTIVE OXYGEN:

- found ≃ 4.60% (determined by adding a known excess of $Na_2SO_3$ in basic medium and by back-titrating iodometrically in acidic medium)
- theoretical = 4.63% (on the basis of 8 active oxygen atoms).

The aqueous phase containing the excess of reactants was recycled into the flask and used again for another preparation, upon the necessary additions (about 1.80 g of $Na_2WO_4$. 2 $H_2O$ dissolved in a small amount of water and neutralized with about 1.8ml of 30% b.w. $H_2SO_4$, 0.35ml of 40% $H_3PO_4$ and about 1ml of 40% $H_2O_2$).

b) Preparation of 3,4-epoxybutyric acid isobutyl ester.

Into a,250ml, four-necked flask provided with blade stirrer, reflux cooler and thermometer, there were introduced, at room temperature, 17ml $H_2O$, 17.04ml of 40% $H_2O_2$ (about 200 mmols), 2.8 g of the catalyst prepared according to example 1a) (about 1 mmol), dissolved in 30ml of dichloroethane and 42.6 g (about 300 mmols) of isobutyl vinylacetate. Under vigorous stirring, the biphasic mixture was heated to 70° C and kept at this temperature for 2.5 hours; a conversion yield based on $H_2O_4$)>99% was obtained (determination by iodometric titration of the aqueous phase). The organic (lower) phase was separated, filtered and, after having evaporated the solvent, quickly pre-distilled under vacuum (6.65-26.6 hPa 5-20 mmHg), what left a residue (4.7 g) containing the catalyst and the heavy products (about 1.9 g). The distilled product was analyzed gas-chromatographically, using a glass column (1.5 m x 2 mmID) packed with 5% Carbowax® 20M on Chromosorb® W "HP" (60-80 mesh) at 75-200° C (10° C/1' ; internal standard: n-hexanol) and was found to contain 26.8 g of epoxide (169.6 mmols) and 16 g of unreacted vinylacetate (112.7 mmols), the epoxide yield (calculated on the charged $H_2O_2$) being thus 84.8% and the epoxide selectivity (calculated on the consumed vinylacetate) being 90.5%. Thereafter the epoxide was separated from vinylacetate by fractional distillation under vacuum (boiling point = 116-118° C at 53hPa (40 mmHg). IR and $^1H$ -NMR spectra of the product were in agreement with the formula, (I) of the epoxide, wherein R was:

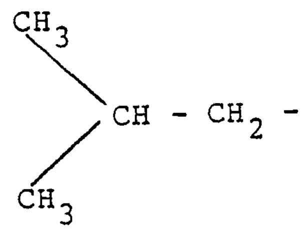

EXAMPLE 2

a), Preparation of the catalyst $(C_{25}H_{54}N)_3PW_4O_{24}$

Example 1 a)was repeated, replacing ARQUAD® 2HT by 1.6 g of methyltrioctylammonium chloride (ALIQUAT® 336), thereby obtaining 2.82 g of a syrup; the analytical data were in agreement with the indicated formula, as shown hereinafter:

ACTIVE OXYGEN:

- found = 5.67% (determined by adding a known excess of $Na_2SO_3$ in basic medium and by back-titrating iodometrically in acidic medium)
- theoretical = 5.68% (on the basis of 8 active oxygen atoms).

b) Preparation of 3,4-epoxybutyric acid isobutyl ester

Example 1 b)was repeated, using the catalyst prepared according to example 2 a) (2.26 g, about 1 mmol), thereby obtaining analogous results [26.86 g (170 mmols) of epoxide; 85% yield (on the charged $H_2O_2$)].

EXAMPLE 3 (Comparative)

10ml of $H_2O$, 6.56ml of 40% $H_2O_2$ (77 millimoles), 1.3 g (0.57 millimoles) of the catalyst prepared according to example 2 a), dissolved in 20ml of benzene, and 20 g (200 millimoles) of the methyl ester of vinylacetate were placed in a 100ml flask provided with blade stirrer, reflux cooler and thermometer. The mixture was heated to 70 °C under vigo rous stirring and kept at said temperature for 2.5 hours ,the organic phase being then treated as in example 1. Obtained were 4.52 g of epoxide (39 millimoles), corresponding to an epoxide yield (calculated on the charged $H_2O_2$) of 50.3%.

## Claims

**1.** Method for the preparation of alkyl esters of 3,4-epoxy-butyric acid of formula:

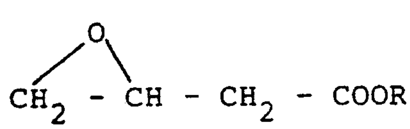

wherein R is a linear or branchend alkyl group containing from 4 to 10 C atoms, wherein the corresponding vinylacetate of formula:

$CH_2=CH\text{-}CH_2\text{-}COOR$

is catalytically oxidized under phase transfer conditions in a biphasic liquid system comprising:

a) an aqueous phase, containing hydrogen peroxide;

b) an organic phase, containing said vinylacetate and a catalyst selected from the peroxidic compounds of formula:

$(R_1R_2R_3R_4N)_3PW_4O_{24}$

as well as, optionally, a solvent immiscible with the aqueous phase, $R_1$, $R_2$, $R_3$ and $R_4$ are equal to each other or different, being hydrocarbon groups altogether having a number of carbon atoms ranging from 20 to 70 and preferably from 25 to 40.

**2.** Method according to claim 1, wherein the catalyst is selected from the peroxidic compounds of the formulae:
$(C_{25}H_{54}N)_3PW_4O_{24}$ and $(C_{37}H_{78}N)_3PW_4O_{24}$.

**3.** Method according to one or both of the claims 1-2, wherein said ester is the isobutyl ester of 3,4-epoxybutyric acid.

**4.** Method according to one or more of the claims 1-3, wherein the oxidation temperature is from 50 to 90 °C, the $H_2O_2$: vinylacetate molar ratio is from 1:1 to 1:2 and the $H_2O_2$: catalyst molar ratio is between 200 and 100.

**5.** Method according to one or more of the claims 1-4, wherein the $H_2O_2$ concentration in the aqueous phase is between 1 % and 70 % and preferably between 10 % and 30 % by weight.

**6.** Method according to one or more of the claims 1-5, wherein the concentration of the vinylacetate in the organic phase, when the operation is carried out in the presence of a solvent immiscible with water, is from 30 % to 90 % and preferably from 50 to 80 % by weight.

**7.** Method according to one or more of the claims 1-6, wherein said solvent is selected from the group consisting of chlorinated aliphatic hydrocarbons and aromatic, optionally substituted, hydrocarbons.

**8.** Method according to one or more of the claims 1-5, wherein the vinylacetate to be oxidized is also used as the solvent for the reaction.

## Revendications

**1.** Procédé de préparation d'esters alkyliques de l'acide 3,4-époxybutyrique, de formule:

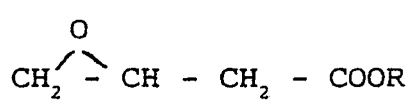

où R est un groupe alkyle à chaîne droite ou ramifiée contenant de 4 à 10 atomes de carbone, où l'acétate de vinyle correspondant, de formule:

$CH_2 = CH - CH_2COOR$

subissant une oxydation catalytique dans les conditions d'un transfert de phase, dans un système liquide biphasique comprenant:

a) une phase aqueuse contenant du péroxyde d'hydrogène;

b) une phase organique contenant ledit acétate de vinyle et un catalyseur choisi parmi les composés peroxydiques de formule:

$(R_1R_2R_3R_4N)_3\ PW_4O_{24}$

et, facultativement, un solvant non miscible avec la phase aqueuse, les radicaux $R_1$, $R_2$, $R_3$ et $R_4$, étant identiques ou différents les uns des autres, et représentant des groupes hydrocarbonés ayant un nombre total d'atomes de carbone compris entre 20 et 70, et de préférence entre 25 et 40.

**2.** Procédé selon la revendication 1, dans lequel le catalyseur est choisi parmi les composés peroxydiques de formules:
$(C_{25}H_{54}N)_3PW_4O_{24}$ et $(C_{37}H_{78}N)_3PW_4O_{24}$

3. Procédé selon l'une ou les deux revendications 1 et 2, dans lequel ledit ester est l'ester isobutylique de l'acide 3,4-époxybutyrique.

4. Procédé selon l'une ou plusieurs des revendications 1 à 3, dans lequel la température d'oxydation est de 50 à 90° C, la proportion molaire $H_2O_2$/acétate de vinyle est de 1/1 à 1/2, et la proportion molaire $H_2O_2$/catalyseur est comprise entre 200 et 100.

5. Procédé selon l'une ou plusieurs des revendications 1 à 4, dans lequel la concentration du $H_2O_2$ dans la phase aqueuse est comprise entre 1 et 70 % et de préférence entre 10 et 30 % en poids.

6. Procédé selon l'une ou plusieurs des revendications 1 à 5, dans lequel la concentration de l'acétate de vinyle dans la phase organique, quand l'opération est mise en oeuvre en présence d'un solvant non miscible avec l'eau, est de 30 à 90 % en poids et de préférence de 50 à 80 % en poids.

7. Procédé selon l'une ou plusieurs des revendications 1 à 6, dans lequel ledit solvant est choisi dans l'ensemble comprenant les hydrocarbures aliphatiques chlorés et les hydrocarbures aromatiques éventuellement substitués.

8. Procédé selon l'une ou plusieurs des revendications 1 à 5, dans lequel l'acétate de vinyle à oxyder est aussi utilisé comme solvant pour la réaction.

**Ansprüche**

1. Verfahren zur Herstellung von Alkylestern der 3,4-Epoxybuttersäure der Formel

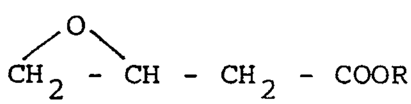

in welcher R eine lineare oder verzweigte Alkylgruppe mit 4 bis 10 Kohlenstoffatomen bedeutet, worin das entsprechende Vinylacetat der Formel
$CH_2 = CH - CH_2 - COOR$
in einem biphasischen flüssigen System unter Phasentransfer-Bedingungen katalytisch oxidiert wird, das umfaßt:
   a) eine wäßrige Phase, die Wasserstoffperoxid enthält;
   b) eine organische Phase, die das Vinylacetat und einen Katalysator, ausgewählt aus den Peroxidverbindungen der Formel
   $(R_1R_2R_3R_4N)_3PW_4O_{24}$
   sowie wahlweise ein mit der wäßrigen Phase nicht mischbares Lösungsmittel enthält, wobei $R_1$, $R_2$, $R_3$ und $R_4$ gleich oder voneinander verschieden sein können und Kohlenwasserstoffgruppen mit insgesamt 20 bis 70 und vorzugsweise 25 bis 40 Kohlenstoffatomen sind.

2. Verfahren gemäß Anspruch 1, in welchem der Katalysator aus den Peroxidverbindungen der Formeln $(C_{25}H_{54}N)_3PW_4O_{24}$ und $(C_{37}H_{78}N)_3PW_4O_{24}$ ausgewählt wird.

3. Verfahren gemäß einem oder beiden der Ansprüche 1 bis 2, in welchem der Ester der Isobutylester von 3,4-Epoxybuttersäure ist.

4. Verfahren gemäß einem oder mehreren der Ansprüche 1 bis 3, in welchem die Oxidationstemperatur 50 bis 90° C beträgt, das Mol-Verhältnis von $H_2O_2$:Vinylacetat zwischen 1:1 bis 1:2 liegt und das Mol-Verhältnis von $H_2O_2$:Katalysator zwischen 200 und 100 liegt.

5. Verfahren gemäß einem oder mehreren der Ansprüche 1 bis 4, in welchem die $H_2O_2$-Konzentration in der wäßrigen Phase zwischen 1 und 70 %, vorzugsweise zwischen 10 und 30 Gew.-%, liegt.

6. Verfahren gemäß einem oder mehreren der Ansprüche 1 bis 5, in welchem die Konzentration des

Vinylacetates in der organischen Phase 30 bis 90 %, vorzugsweise 50 bis 80 Gew.-%, beträgt, wenn das Verfahren in Gegenwart eines mit Wasser nicht mischbaren Lösungsmittels durchgeführt wird.

7. Verfahren gemäß einem oder mehreren der Ansprüche 1 bis 6, in welchem das Lösungsmittel ausgewählt wird aus chlorierten aliphatischen Kohlenwasserstoffen und aromatischen, wahlweise substituierten, Kohlenwasserstoffen.

8. Verfahren gemäß einem oder mehreren der Ansprüche 1 bis 5, in welchem das zu oxidierende Vinylacetat gleichzeitig als Lösungsmittel für die Reaktion verwendet wird.